# EUROPEAN PATENT SPECIFICATION

(11) **EP 1 091 729 B1**
(45) Date of publication and mention of the grant of the patent: **02.04.2008**
(21) Application number: 99928111.6
(22) Date of filing: 28.06.1999
(51) Int. Cl.: A61K 9/00, A61K 9/16, A61K 38/28, A61K 38/46, A61K 38/48, A61K 38/06

(54) **SPRAY-DRIED MICROPARTICLES OF INSULIN FOR INHALATION**
SPRÜHGETROCKNETE INSULIN-MIKROPARTIKEL ZUR INHALATION
MICROPARTICULES D'INSULINE SéCHéES PAR ATOMISATION POUR INHALATION

(30) Priority: 30.06.1998 GB 9814172
(43) Date of publication of application: 18.04.2001
(73) Proprietor: Quadrant Drug Delivery Limited, Nottingham, NG11 6JS (GB)
(72) Inventor: ROBINSON, Stuart, Oakham Rutland LE15 7LE (GB); SMITH, Susan, Stewart, Loughborough Leicestershire LE77 0NF (GB)
(74) Representative: Jappy, John William Graham
(86) International application number: PCT/GB1999/002023
(87) International publication number: WO 2000/000176

(56) References cited:
- EP-A- 0 542 314
- WO-A-95/23613
- WO-A-95/24183
- DE-B- 1 227 855
- US-A- 3 515 642
- YEO, SANG-DO ET AL.: "Formation of Microparticulate Protein Powders Using a Supercritical Fluid Antisolvent" BIOTECHNOLOGY AND BIOENGINEERING, vol. 41, no. 3, 5 February 1993 (1993-02-05), pages 341-346, XP002116795 ISSN: 0006-3592
- FORBES R T ET AL: "WATER VAPOR SORPTION STUDIES ON THE PHYSICAL STABILITY OF A SERIES OF SPRAY-DRIED PROTEIN/SUGAR POWDERS FOR INHALATION" JOURNAL OF PHARMACEUTICAL SCIENCES, vol. 87, no. 11, 1 November 1998 (1998-11-01), pages 1316-1321, XP000783384 ISSN: 0022-3549
- CHEMICAL ABSTRACTS, vol. 93, no. 2, 14 July 1980 (1980-07-14) Columbus, Ohio, US; abstract no. 13123, VEBER, PAVEL ET AL: "Preparations releasing cells from animal tissues or surfaces of cultivation vessels" XP002116796 & CS 180 896 B (CZECH.) 15 September 1979 (1979-09-15)

## Description

### Field of the Invention

This invention relates to a formulation of a therapeutic agent such as insulin, that is suitable for administration to the lung, and that has good stability.

### Background of the Invention

There is now widespread interest in the formulation of therapeutic agents for inhalation. In particular, many efforts have been made to formulate suitable therapeutic agents as dry powders for delivery via inhalers.

Typically, the formulations are produced by drying the active agent in the presence of certain excipients, such as polysaccharides or citrate, to enhance stability during the drying process or in storage.

Insulin is a typical example of a therapeutic agent that can be administered to the lung, by inhalation. As a commercial product, insulin is generally provided in suspension or a solution of low concentration, as a hexamer complexed with zinc. Refrigeration is necessary, in order to maintain the stability of such a formulation. Crystalline Zn insulin is stable at neutral pH. The dry powder also requires refrigeration.

CA-A-2136704 discloses a product obtained by spray-drying a medicinal substance such as insulin (among many others) and a carrier. Example 4 discloses spray-drying a clear solution of human insulin, soya bean lecithin and lactose.

WO-A-9735562 again discloses spray-drying a solution of insulin and a polysaccharide. The aim of this combination is to achieve the preferred size range of spray-dried microparticles, for good lung deposition. In Examples 1 and 3, the insulin solution for spray-drying, prior to combination with polysaccharide, is prepared by dissolving zinc insulin in HCl, and then adding NaOH, to pH 7.2. The solutions for spray-drying respectively contain 25 and 6 mg/ml insulin and at least 5.5/7.2% NaCl, based on the combined weight of insulin plus salt.

WO-A-9524183 is directed primarily to a dry powder that comprises insulin and a carrier material, typically a saccharide, in the form of an amorphous powder of microparticles obtained by spray-drying. In addition, the Experimental section compares the properties of such microparticles with and without a saccharide excipient. The insulin solution for spray-drying is prepared by dissolving Zn-insulin in citrate buffer, to pH 6.7 ± 0.3, to a solids content of 7.5 mg/ml. The powder is held in a container at 10% RH. The formulation without saccharide has considerably lower bioavailability than those with saccharide. For various reasons, this experiment cannot be reproduced: citrate is a buffer at pH 3.0-6.2, and not at pH 6.7; crystalline insulin will not dissolve in pH 6.2 citrate buffer before or after adjustment to pH 7.4 with NaOH; in any case, no alkali addition is specified.

The presence of citrate in dry powder formulations was believed to be necessary to enhance the stability of the final product (Drug Development and Industrial Pharmacy 1984; 10(3):425-451). However, in many cases, the high citrate concentration dilutes the amount of active agent in the initial feedstock, resulting in low amounts of active for drying.

Pikal and Rigsbee, Pharm. Rev. 14(10):1379-87 (1997), report that freeze-dried amorphous insulin was significantly more stable than crystalline insulin at corresponding water contents from 0 to 15% w/w. The mechanism was unclear, but may have been due to configurational differences between the amorphous and crystalline states, the reactive parts of the protein being in closer proximity in the latter. The formulation reported by Pikal and Rigsbee contains no salt, because such low concentrations of insulin (c. 0.5% w/v) are used that manipulation of the pH is unnecessary.

WO95/23613 discloses a spray-dried DNase formulation. The spray-dried product is in a crystalline form, due primarily to a high concentration of salt. It is stated that high concentrations of salt increases the dispersibility qualities of the final product. In Example 1, the final product contains 60% salt compared with 30% of the DNase.

In summary, the prior art discloses various results of interest but of uncertain commercial significance. None of the procedures described above gives a pure insulin product that is stable, or uses a sufficiently concentrated solution for spray-drying, to be suitable as a commercial procedure. The most effective procedures invariably suggest that co-spray-drying of insulin and, say, a saccharide is necessary for best results.

### Summary of the Invention

The present invention is based on the surprising discovery that it is possible to spray-dry insulin at higher (and therefore commercially useful) concentrations than have been used previously, without the concomitant production of an undesirable high concentration of salt or other excipients. Such formulations show no substantial loss of activity after the drying process and have extended stability, by comparison with pre-spray-dried preparations.

According to the present invention microparticies, obtainable by spray-drying a substantially pure solution of insulin, consists of insulin. In a preferred embodiment, the microparticles consists only of insulin and NaC 1 salt. Such microparticles may be held in a container at greater than 10% RH, and thus essentially at ambient humidity. The insulin microparticles are obtainable by dissolving Zn-insulin in acid, adding alkali to give an insulin solution, e.g. to a pH above 7, and spray-drying the insulin solution (which also contains a salt formed as a result of the dissolution process, or a buffer).

Preferably, the microparticles are non-crystalline/amorphous.

### Description of the Invention

As indicated above, microparticles of the invention "consist" of insulin. This term is used herein to indicate that they are substantially free of polysaccharide, or buffer salt, e.g citrate, since none is necessary. The absence of polysaccharide has the advantage that a given unit dosage, e.g. a particle, contains essentially only the intended active component. This is an important consideration, for a drug that may be required in large amounts. Another advantage is the avoidance of delivering unnecessary material to a subject. A further advantage is that consistent dosing of the therapeutic agent is facilitated; this is especially important where there is a narrow therapeutic window.

The absence of buffer salt is desirable as it allows a more concentrated feedstock solution of the insulin to be spray-dried resulting in significant cost savings and providing a more commercial-scale process to be adopted.

The term "substantially pure" is used herein to indicate that the feedstock solution to be spray-dried comprises primarily only insulin and solvent. There may be a minor amount of solids other than the insulin, but this has no significant effect on the eventual stability of the product.

Insulin microparticles of the invention may include components that are produced during the successive addition of acid and alkali, in preparation of the feedstock, e.g. a salt. For example, NaCl is formed if the acid and alkali are respectively HCl and NaOH. It has been found that the presence of NaCl apparently has no stabilising effect. Indeed, stability may be greater with reduced amounts of salt, again allowing a more concentrated feedstock to be used.

Typically, the solution for spray-drying may contain less than 4% by weight of salt, by weight of total solids. The salt content is based on theoretical considerations, by titration to pH 7. More particularly, this value is calculated by consideration of the molar quantities of the ions added during dissolution. The solution may contain any desired amount of the therapeutic agent, e.g. more than 20, 30 or 50 mg/ml, often up to 100 or 200 mg/ml.

As indicated above, successive addition of acid and alkali apparently destroys the crystalline form of Zn insulin. Zn may dissociate from the hexameric complex but need not be removed. Accordingly, Zn may be present in the microparticles. If desired, this or any other component, other than the therapeutic agent, may be removed, using any suitable technique known to those of skill in the art. In a preferred embodiment for insulin, the Zn is removed from solution prior to spray-drying. This may be achieved by diafiltering the solution according to methods known in the art. The Zn-free insulin may have greater stability than the Zn-containing product. Moisture may also be present.

As disclosed in more detail in WO-A-9218164, WO-A-9408627 and other Andaris publications, the conditions of spray-drying can be controlled so that microparticles having a defined size range, e.g. 0.1 to 50 µm, can be obtained. The mass median particle size is preferably 1 to 10 µm, when the product is intended for administration by inhalation.

The microparticles (microcapsules) obtained by spray-drying may be solid or hollow. Further, the surface may be smooth or "dimpled"; a dimpled surface may be beneficial for inhalation.

The microparticles have good stability and may be maintained as such, i.e. as a dry powder, in a container. During storage or in formulation, they may be mixed with any suitable pharmaceutical agents, carriers, bulking agents etc, and they may be processed by any technique desired to give a product having the properties intended for the ultimate therapeutic use. In particular, the formulation of particles for formulations that can be delivered to the lung, e.g. using a metered dose or dry powder inhaler, are known to those skilled in the art.

The nature of the container is not critical. For example, it may be a glass jar or plastics box. It merely defines a storage environment within which, unlike the prior art and as evidenced below, there is no need to remove moisture or otherwise to control the conditions.

Insulin that is used in the invention may be of any suitable form. It may be, for example, bovine or human insulin. Results that have been obtained, regarding the stability of bovine insulin, apparently apply also to human insulin.

The following Examples illustrate the invention.

### Example 1

A solution of bovine or human insulin for spray-drying is typically prepared in the following way: 5 g insulin is dissolved in 70 ml 0.05 m HCl, after which the solution is back-titrated with sufficient 1M NaOH to reform a solution from the isoelectric point precipitate. According to the final concentration required, water is added to make to volume. Approximately 4.8 ml 1M NaOH is required, in this Example. The solution is then spray-dried using a Mini spray drier with an outlet temperature of approximately 87°C and a solution feed rate of approximately 0.75 g/min.

Reverse Phase High Performance Liquid Chromatography (RP-HPLC) was used to assess the stability of insulin, under the following conditions:

| | |
|---|---|
| Column: | Vydac C-18, 5µm, 30 nm |
| Mobile Phase: | A- 0.1%TFA in water |
| | B- 0.1% TFA in acetonitrile (95%) and water (5%) |
| | Gradient Elution |
| Flow Rate: | 1.5mL/min |
| Detection: | UV at 220nm |
| Injection Volume: | 100µL |

Under these conditions, bovine insulin has a retention time of approximately 7.4 minutes.

A peak attributable to deamidated insulin is located at the tailing edge of the main peak. The extent of deamidation is used to indicate stability and is calculated by expressing the area of the deamidation peak as a percentage of the total peak area. Total degradation is expressed as the area of all degradant peaks as a percentage of the total peak area.

**Table 1**

| Percentage Total Degradation and Deamidation of Non-Spray Dried Bovine Crystalline Insulin | | | | |
|---|---|---|---|---|
| Time | 2°C/Ambient RH | | 30°C/60% RH | |
| | % Deamidation | % Degradation | % Deamidation | % Degradation |
| Initial | 3.2 | 3.6 | 3.2 | 3.6 |
| 1 month | 3.5 | 3.9 | 4.2 | 5.3 |
| 3 months | 4.4 | 5.6 | 5.8 | 11.0 |
| 6 months | 3.4 | 4.5 | 6.7 | 13.7 |

**Table 2**

| Percentage Total Degradation and Deamidation of Insulin Microparticles | | | | |
|---|---|---|---|---|
| Time | 2°C/Ambient RH | | 30°C/60% RH | |
| | % Deamidation | % Degradation | % Deamidation | % Degradation |
| Initial | 2.4 | 3.1 | 2.4 | 3.1 |
| 1 month | 2.8 | 3.9 | 3.1 | 4.6 |
| 3 months | 2.0 | 2.7 | 2.6 | 4.1 |
| 6 months | 1.9 | 3.0 | 2.8 | 5.0 |

The results indicate that the extent of both deamidation and total degradation is increased with time, for all the batches evaluated. Additionally, the data suggest that spray drying appears to confer additional stability to the protein, in that the bovine crystalline insulin control suffers increased degradation in comparison to the microparticle formulations at comparable timepoints after storage at 30°C/60% RH.

To investigate whether this was also seen with human insulin, human insulin microparticles were prepared (by the same general procedure as that described above) and placed on accelerated stability at 40°C/75% RH. All samples were analysed by RP-HPLC at the initial time point, and also after 1 week, 2 weeks and 5 weeks.

**Table 3**

| Effect of Storage at 40°C/75% RH on the Deamidation and Total Degradation Levels of Human Insulin Recombinant From E. Coli | | |
|---|---|---|
| Storage Time at 40°C/75% RH | % Deamidation | % Total Degradation |
| 0 | 0.59 | 0.75 |
| 1 | 1.57 | 4.27 |
| 2 | 1.62 | 5.15 |
| 5 | 2.37 | 8.40 |

**Table 4**

| Effect of Storage at 40°C/75% RH on the Deamidation and Total Degradation Levels of Human Insulin Microparticle | | |
|---|---|---|
| Storage Time at 40°C/75% RH | % Deamidation | % Total Degradation |
| 0 | 0.95 | 1.65 |
| 1 | 1.08 | 2.20 |
| 2 | 1.03 | 3.10 |
| 5 | 1.21 | 3.32 |

Comparing Tables 3 and 4, the microparticle formulation of human insulin is less prone to degradation, showing only 3.32% total degradation after 5 weeks at 40°C/75% RH compared to 8.40% total degradation for the material stored as received under the same conditions.

## Claims

1. Spray-dried microparticles consisting of insulin and optionally a salt present at less than 4% by weight of total solids.

2. Microparticles according to claim 1, wherein the insulin is non-crystalline/amorphous.

3. A closed container containing, at ambient humidity, microparticles according to claim 1 or claim 2.

4. An inhaler device comprising microparticles according to any of claim 1 or claim 2.

5. A process for the preparation of microparticles according to any of claims 1 or 2, which comprises spray-drying a substantially pure solution of insulin.

6. A process according to claim 5, wherein the solution contains more than 10 mg/ml of insulin.

7. A process according to claim 6, wherein the solution contains 20 to 200 mg/ml insulin.

8. A process according to claim 6, wherein the solution contains 50 to 100 mg/ml insulin.

9. A process according to any of claims 5 to 8, wherein the solution is free of buffer salt.

10. A process according to any of claims 5 to 9, wherein the solution is obtainable by dissolving insulin in acid, and then adding alkali.

11. A process according to claim 10, wherein the insulin is Zn-insulin.

12. A process according to claim 11, wherein the Zn is removed from solution prior to spray-drying.

## Patentansprüche

1. Sprühgetrocknete Mikropartikel, bestehend aus Insulin und gegebenenfalls einem Salz, vorliegend weniger als 4 Gew.-% des Gesamtfeststoffs.

2. Mikropartikel nach Anspruch 1, wobei das Insulin nicht-kristallin/amorph ist.

3. Geschlossener Behälter, der bei Umgebungsfeuchte Mikropartikel nach Anspruch 1 oder Anspruch 2 enthält.

4. Inhalatorvorrichtung, die Mikropartikel nach Anspruch 1 oder Anspruch 2 umfasst.

5. Verfahren zur Herstellung von Mikropartikeln nach Anspruch 1 oder Anspruch 2, welches das Sprühtrocknen einer im Wesentlichen reinen Lösung von Insulin umfasst.

6. Verfahren nach Anspruch 5, wobei die Lösung mehr als 10 mg/ml Insulin enthält.

7. Verfahren nach Anspruch 6, wobei die Lösung 20 bis 200 mg/ml Insulin enthält.

8. Verfahren nach Anspruch 6, wobei die Lösung 50 bis 100 mg/ml Insulin enthält.

9. Verfahren nach einem der Ansprüche 5 bis 8, wobei die Lösung frei von Puffersalz ist.

10. Verfahren nach einem der Ansprüche 5 bis 9, wobei die Lösung durch Lösen von Insulin in Säure und dann Zugabe von Alkali erhältlich ist.

11. Verfahren nach Anspruch 10, wobei das Insulin Zn-Insulin ist.

12. Verfahren nach Anspruch 11, wobei das Zn vor der Sprühtrocknung aus der Lösung entfernt wird.

## Revendications

1. Microparticules séchées par pulvérisation consistant en insuline et éventuellement un sel présent à raison de moins de 4 % en masse des solides totaux.

2. Microparticules selon la revendication 1, où l'insuline est non cristalline/amorphe.

3. Récipient fermé contenant, à l'humidité ambiante, des microparticules selon la revendication 1 ou la revendication 2.

4. Dispositif inhalateur comprenant des microparticules selon l'une quelconque de la revendication 1 ou de la revendication 2.

5. Procédé pour la préparation de microparticules selon l'une quelconque des revendications 1 ou 2 qui comprend le séchage par pulvérisation d'une solution sensiblement pure d'insuline.

6. Procédé selon la revendication 5, où la solution contient plus de 10 mg/ml d'insuline.

7. Procédé selon la revendication 6, où la solution contient 20 à 200 mg/ml d'insuline.

8. Procédé selon la revendication 6, où la solution contient 50 à 100 mg/ml d'insuline.

9. Procédé selon l'une quelconque des revendications 5 à 8, où la solution est dépourvue de sel tampon.

10. Procédé selon l'une quelconque des revendications 5 à 9, où la solution peut être obtenue en dissolvant l'insuline dans un acide puis en ajoutant un alcali.

11. Procédé selon la revendication 10, où l'insuline est la Zn-insuline.

12. Procédé selon la revendication 11, où le Zn est retiré de la solution avant le séchage par pulvérisation.
